# EUROPÄISCHE PATENTSCHRIFT

(11) **EP 1 282 586 B1**
(45) Veröffentlichungstag und Bekanntmachung des Hinweises auf die Patenterteilung: **10.01.2007**
(21) Anmeldenummer: 01945105.3
(22) Anmeldetag: 09.05.2001
(51) Int. Cl.: C07C 11/167, C07C 7/163, C07C 7/08, B01D 3/40

(54) **VEFAHREN UND VORRICHTUNG ZUR AUFARBEITUNG EINES C4-SCHNITTES**
METHOD AND DEVICE FOR TREATING A C4 FRACTION
PROCEDE ET DISPOSITIF POUR TRAITER UNE FRACTION C4

(30) Priorität: 09.05.2000 DE 10022465
(43) Veröffentlichungstag der Anmeldung: 12.02.2003
(73) Patentinhaber: BASF Aktiengesellschaft, 67056 Ludwigshafen (DE)
(72) Erfinder: MEYER, Gerald, 67067 Ludwigshafen (DE); KAIBEL, Gerd, 68623 Lampertheim (DE); BOHNER, Gerd, 69254 Malsch (DE); KINDLER, Klaus, 67376 Harthausen (DE); ADRIAN, Till, 67240 Bobenheim-Roxheim (DE); PICKENÄCKER, Karin, 68623 Lampertheim (DE); PAHL, Melanie, 68167 Mannheim (DE); HILL, Thomas, 67051 Ludwigshafen (DE)
(74) Vertreter: Isenbruck, Günter
(86) Internationale Anmeldenummer: PCT/EP2001/005279
(87) Internationale Veröffentlichungsnummer: WO 2001/085656

(56) Entgegenhaltungen:
- US-A- 4 277 313
- US-A- 6 040 489

## Beschreibung

Die Erfindung betrifft ein Verfahren zur Gewinnung von 1,3-Butadien durch Aufarbeitung eines C₄-Schnitts sowie eine Vorrichtung zur Durchführung des Verfahrens. Der in Crackern anfallende sogenannte C₄-Schnitt umfaßt ein Gemisch von Kohlenwasserstoffen, wobei die C₄-Kohlenwasserstoffe, insbesondere 1-Buten, i-Buten sowie 1,3-Butadien überwiegen. Neben geringen Mengen an C₃- und C₅-Kohlenwasserstoffen enthält der C₄-Schnitt in der Regel C₃- und C₄-Acetylene, z.B. 1-Butin, Butenin und Propin, insbesondere 1-Butin (Ethylacetylen) und Butenin (Vinylacetylen).

Die Gewinnung von 1,3-Butadien aus derartigen Gemischen ist wegen der geringen Unterschiede in den relativen Flüchtigkeiten ein kompliziertes destillationstechnisches Problem. Daher wird die Auftrennung durch eine sogenannte Extraktivdestillation durchgeführt, das heißt eine Destillation unter Zugabe eines Extraktionsmittels, das einen höheren Siedepunkt als das aufzutrennende Gemisch aufweist und das die Unterschiede in den relativen Flüchtigkeiten der aufzutrennenden Komponenten erhöht. Durch Einsatz geeigneter Extraktionsmittel kann aus dem genannten C₄-Schnitt mittels Extraktivdestillation eine Roh-1,3-Butadienfraktion erhalten werden, die anschließend in Reindestillationskolonnen weitergereinigt wird, neben einem Strom, der die weniger als 1,3-Butadien löslichen Kohlenwasserstoffe, insbesondere Butane und Butene (Raffinat 1) enthält, sowie einem Strom, der die leichter als 1,3-Butadien löslichen Kohlenwasserstoffe, insbesondere die Butine sowie gegebenenfalls 1,2-Butadien enthält. Ein derartiges Verfahren ist beispielsweise in EP-B 0 284 971 beschrieben. Es hat jedoch den Nachteil, daß die im Extraktionsmittel besser als 1,3-Butadien löslichen Komponenten, insbesondere die Butenine sowie gegebenenfalls 1,2-Butadien nicht in das Wertprodukt 1,3-Butadien überführt werden.

Als weiterer Nachteil ist ein Raffinatverlust zu verzeichnen, da der acetylenreiche Strom aus sicherheitstechnischen Gründen mit Raffinat 1 verdünnt werden muß.

Die Extraktivdestillation zur Gewinnung von 1,3-Butadien kann durch eine vorgeschaltete Selektivhydrierung acetylenischer Verunreinigungen, das heißt der Butine, vereinfacht werden. Ein derartiges Verfahren ist in Proc.-Ethylene Prod. Conf. 5 (1996), Seiten 631 bis 636 beschrieben. Danach wird unter Verwendung eines sogenannten KLP-Katalysators, das heißt einem Katalysator, der feinverteilte Kupfer-Partikel auf einem hochreinen γ-Aluminiumoxid als Träger mit definierter Porenstruktur enthält, ein hoher Vinylacetylenumsatz bei niedrigem Butadienverlust erreicht, bei hohen Katalysatorstandzeiten. Durch die vorgeschaltete Selektivhydrierung kann die zweistufige Butadien-Extraktivdestillation auf einen Einstufenprozeß vereinfacht sowie der apparative Aufwand in der nachgeschalteten Reindestillation um eine Trennkolonne reduziert werden. Das Verfahren hat jedoch den Nachteil, daß eine separate Anlage zur vorgeschalteten Selektivhydrierung der acetylenischen Verunreinigungen erforderlich ist.

Aus der US 4,277,313 ist ein weiteres Verfahren zur Gewinnung von 1,3-Butadien bekannt, wonach zunächst eine Selektivhydrierung und anschließend eine Extraktivdestillation des 1,3-Butadiens durchgeführt wird. Die Selektivhydrierung kann in Flüssig- oder Gasphase, in Gegenwart von Katalysatoren der VIII. Gruppe des Periodensystems, beispielsweise an einem Palladium/Aluminiumoxid-Katalysator durchgeführt werden. Als Extraktionsmittel werden Dimethyl- oder Diethylformamid, N-Methylpyrrolidon, Furfurol oder Acetonitril genannt. Das Verfahren weist, analog zum vorstehend beschriebenen, den Nachteil auf, daß für die vorgeschaltete Selektivhydrierung eine getrennte Anlage erforderlich ist.

Aus US 6,040,489 ist ein Verfahren zur Abtrennung von 1,3-Butadien aus einem C₄-Schnitt bekannt, wobei der C₄-Schnitt in einer Kolonne hydriert und mit einem Lösungsmittel selektiv extrahiert wird, aus der Kolonne ein mindestens die Butane und Butene umfassender Strom als Kopfstrom abgezogen und das mit Butadienen beladene Lösungsmittel über Sumpf abgezogen und anschließend in einer Lösungsmittel-Strippkolonne in einen butadienhaltigen Kopfstrom und einen lösungsmittelhaltigen Sumpfstrom aufgetrennt wird. Der butadienhaltige Kopfstrom wird in einer Butadien-Destillationskolonne in einen 1,3-butadienhaltigen Kopfstrom und einen 1,2-butadienhaltigen Sumpfstrom aufgetrennt.

Zur destillativen Auftrennung von Mehrkomponentengemischen sind sogenannte Trennwandkolonnen bekannt, das heißt Destillationskolonnen mit senkrechten Trennwänden, die in Teilbereichen eine Quervermischung von Flüssigkeits- und Brüdenströmen verhindern. Die Trennwand, die aus einem ebenen Blech besteht, unterteilt die Kolonne in Längsrichtung in deren mittleren Bereich in einen Zulaufteil und einen Entnahmeteil.

Ein ähnliches Ergebnis kann mit sogenannten thermisch gekoppelten Kolonnen erreicht werden, das heißt Anordnungen von mindestens zwei Kolonnen, wobei jede der Kolonnen mit jeder anderen mindestens zwei Verknüpfungen an räumlich getrennten Stellen aufweist.

Die. EP-B 0 126 288 beschreibt eine Trennwandkolonne, in der chemische Reaktionen durchgeführt werden. Durch definierte Zugabe von homogenen Katalysatoren lassen sich danach chemische Reaktionen gezielt auf bestimmte Teilbereiche der Trennwandkolonne eingrenzen.

Es ist Aufgabe der Erfindung, ein Verfahren zur Gewinnung von 1,3-Butadien aus einem C₄-Schnitt zur Verfügung zu stellen, das die Nachteile des Standes der Technik nicht ausweist, insbesondere einen geringeren apparativen Aufwand erfordert.

Vorliegend wird als Roh-1,3-Butadien ein Kohlenwasserstoffgemisch bezeichnet, das das Wertprodukt 1,3-Butadien in einem Anteil von mindestens 80 Gew.-%, bevorzugt 90 Gew.%, besonders bevorzugt 95 Gew.-%, Rest Verunreinigungen, enthält.

Dem gegenüber wird als Rein-1,3-Butadien ein Kohlenwasserstoffgemisch bezeichnet, das das Wertprodukt 1,3-Butadien in einem Anteil von mindestens 99 Gew.-%, bevorzugt 99,5 Gew.-%, besonders bevorzugt 99,7 Gew.-%, Rest Verunreinigungen enthält.

Die Lösung geht aus von einem Verfahren zur Aufarbeitung eines C₄-Schnittes, umfassend die Verfahrensschritte
- Extraktivdestillation (I),
- Selektivhydrierung an einem heterogenen Katalysator (II), wobei nach den Verfahrensschritten (I) und (II) ein Roh-1,3-Butadienstrom erhalten wird und
- Destillation des Roh-1,3-Butadienstromes zur Gewinnung von Rein-1,3-Butadien (III).

Die Lösung ist dadurch gekennzeichnet, daß die Verfahrensschritte I und II in einer einzigen Kolonne und der Verfahrensschritt III in einer zweiten Kolonne durchgeführt wird.

Alternativ ist es möglich, die Verfahrensschritte I und II in thermisch gekoppelten Kolonnen und den Verfahrensschritt III in einer zweiten Kolonne durchzuführen.

Aus den bekannten Verfahren gibt es keine Anhaltspunkte dafür, daß man einen C₄-Schnitt durch Extraktivdestillation und Selektivhydrierung in heterogener Katalyse unter Erhalt eines Roh-1,3-Butadienstromes in einer einzigen Kolonne durchführen könnte. Man ging im Gegenteil davon aus, daß zur Abtrennung von 1,3-Butadien aus dem damit beladenen selektiven Lösungsmittel ein zusätzlicher Apparat, insbesondere eine Strippkolonne erforderlich sei, die unter anderen Verfahrensbedingungen, insbesondere unter anderen Druckbedingungen betrieben werden müsse. Der Grund hierfür liegt in der starken Polymerisationsneigung der dienischen sowie der acetylenischen Verbindungen bei erhöhter Temperatur. Diese Temperaturerhöhungen treten im unteren Kolonnenbereich sowie im Verdampfer auf, wenn die leichtsiedenden Kohlenwasserstoffe aus dem hochsiedenden Extraktionsmittel destillativ unter den Druckbedingungen der Extraktivdestillation, d.h. bei etwa 4 bis 6 bar absolut, abgetrennt werden.

Der vorliegend als Ausgangsgemisch einzusetzende sogenannte C₄-Schnitt ist ein Gemisch von Kohlenwasserstoffen mit überwiegend vier Kohlenstoffatomen pro Molekül. C₄-Schnitte werden beispielsweise bei der Herstellung von Ethylen und/oder Propylen durch thermisches Spalten einer Petroleumfraktion wie verflüssigtes Petroleumgas, Leichtbenzin oder Gasöl erhalten. Weiterhin werden C₄-Schnitte bei der katalytischen Dehydrierung von n-Butan und/oder n-Buten erhalten. C₄-Schnitte enthalten in der Regel Butane, Butene, 1,3-Butadien, daneben geringe Mengen an C₃- und C₅-Kohlenwasserstoffen, sowie Butine, insbesondere 1-Butin (Ethylacetylen) und Butenin(Vinylacetylen). Dabei beträgt der 1,3-Butadiengehalt im allgemeinen 10 bis 80 Gew.-%, vorzugsweise 20 bis 70 Gew.-%, insbesondere 30 bis 60 Gew.-%, während der Gehalt an Vinylacetylen und Ethylacetylen im allgemeinen 5 Gew.-% nicht übersteigt.

Ein typischer C₄-Schnitt weist die folgende Zusammensetzung in Gewichtsprozenten auf:

| | |
|---|---|
| Propan | 0 - 0,5 |
| Propen | 0 - 0,5 |
| Propadien | 0 - 0,5 |
| Propin | 0 - 0,5 |
| n-Butan | 3 - 10 |
| i-Butan | 1-3 |
| 1-Buten | 10 - 20 |
| i-Buten | 10 - 30 |
| trans-2-Buten | 2 - 8 |
| cis-2-Buten | 2 - 6 |
| 1,3-Butadien | 30 - 60 |
| 1,2-Butadien | 0,1-1 |
| Ethylacetylen | 0,1-2 |
| Vinylacetylen | 0,1- 3 |
| C5 | 0 - 0,5 |

Für die eingangs bereits definierte Extraktivdestillation kommen für das vorliegende Trennproblem, der Gewinnung von 1,3-Butadien aus dem C₄-Schnitt, als selektive Lösungsmittel generell Substanzen oder Gemische in Frage, die einen höheren Siedepunkt als das aufzutrennende Gemisch sowie eine größere Affinität zu konjugierten Doppelbindungen und Dreifachbindungen als zu einfachen Doppelbindungen sowie Einfachbindungen aufweisen, bevorzugt dipolare, besonders bevorzugt dipolar-aprotische Lösungsmittel. Aus apparatetechnischen Gründen werden wenig oder nicht korrosive Substanzen bevorzugt.

Geeignete selektive Lösungsmittel für das erfindungsgemäße Verfahren sind zum Beispiel Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im allgemeinen werden N-alkylsubstituierte niedere aliphatische Säureamide oder N-alkylsubstituierte cyclische Säureamide verwendet. Besonders vorteilhaft sind Dimethylformamid und insbesondere N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Colösungsmitteln wie Wasser und/oder tert.-Butylether, zum Beispiel Methyl-tert.-butylether, Ethyl-tert.-butylether, Propyl-tert.-butylether, n- oder iso-Butyl-tert.-butylether eingesetzt werden.

Besonders geeignet ist N-Methylpyrrolidon, bevorzugt in wässriger Lösung, insbesondere mit 8 bis 10 Gew.-% Wasser, besonders bevorzugt mit 8,3 Gew.-% Wasser.

Für die Selektivhydrierung in heterogener Katalyse, dem Verfahrensschritt II, kommen vorliegend grundsätzlich alle bekannten Verfahren in Frage. Es ist möglich, die bekannten Katalysatoren auf der Basis von Palladium, wie sie beispielsweise in EP-A-0 738 540, EP-A-0 722 776 oder US 4,587,369 beschrieben sind, einzusetzen oder Katalysatoren auf der Basis von Kupfer, wie beispielsweise in US 4,493,906 oder US 4,704,492 beschrieben.

Die Katalysatoren für die Selektivhydrierung können auf üblichen destillativen Einbauten, das heißt insbesondere Kolonnenböden, Formkörpern oder Packungen aufgebracht sein, sie können in Taschen von Drahtnetzen eingebettet und Ballen aufgerollt sein, wie in US 4,215,011 beschrieben. Sie können jedoch besonders vorteilhaft als sogenannte TLC (Thin Layer Catalyst)-Packungen eingesetzt werden.

Besonders geeignet sind die in DE-A 196 24 130 beschriebenen durch Aufdampfen und/oder Sputtern erhaltenen TLC-Katalysatorpackungen. Zusätzlich zu den in DE-A 196 24 130 aufgeführten Trägermaterialformen Gewebe oder Folie ist es möglich, als Trägermaterial für die Katalysatorpackung ein Gewirk oder ein Gestrick einzusetzen. Die Aufbringung der katalytisch und/oder als Promotor aktiven Substanz kann außer durch das in DE-A 196 24 130 beschriebene Aufdampfen und/oder Sputtern auch durch Tränkung erreicht werden.

Die Destillation des Roh-1,3-Butadienstromes zur Gewinnung von Rein-1,3-Butadien (Verfahrensschritt III) erfolgt in einer zweiten Destillationskolonne, in bekannter Weise, insbesondere in einer Trennwandkolonne oder in einer Kolonne oder in 2 Kolonnen. Der Feed-Strom für den Verfahrensschritt III wird bevorzugt als dampfförmiger Seitenstrom aus der ersten Kolonne entnommen und der zweiten Destillationskolonne zugeführt.

Bezüglich der zur Durchführung der Verfahrensschritte I und II unter Erhalt eines Roh-1,3-Butadienstromes einsetzbaren Kolonnen gibt es grundsätzlich keine Einschränkungen.

Der Kolonne wird in ihrem mittleren Bereich der C₄-Schnitt zugeführt, das selektive Lösungsmittel in ihrem oberen Bereich und Wasserstoff unterhalb der Zuführung des C₄-Schnitts.

Die Kolonne ist mit trennwirksamen Einbauten bestückt, die bevorzugt im Bereich unterhalb der Zuführung des selektiven Lösungsmittels Füllkörper oder geordnete Packungen sind. Oberhalb der Zuführung des selektiven Lösungsmittels sind bevorzugt ein oder mehrere Böden angeordnet.

Die Kolonne wird bevorzugt bei einem Kopfdruck im Bereich von 3 bis 7 bar absolut, insbesondere von 4 bis 6 bar absolut betrieben; dadurch ist es möglich, mit Wasser als Kühlmittel am Kopf der Kolonne zu kondensieren, aufwendigere Kühlmittel sind nicht erforderlich.

Im Kolonnensumpf stellen sich dabei Temperaturen im Bereich von etwa 140 bis 200°C, insbesondere von 180 bis 190°C, häufig von etwa 185 °C ein.

Zumindest die trennwirksamen Einbauten unterhalb der Zuführung des C₄-Schnitts, insbesondere Füllkörper oder geordnete Packungen, sind als reaktive Einbauten ausgestaltet, d.h. es wurden darauf, wie vorstehend bereits beschrieben, Katalysatoren für die Selektivhydrierung aufgebracht. Bevorzugt werden sogenannte TLC-Packungen eingesetzt.

Am Kolonnenkopf wird ein Strom abgezogen, der die im selektiven Lösungsmittel weniger als 1,3-Butadien löslichen Komponenten des C₄-Schnitts enthält, insbesondere Butane und Butene und aus dem Kolonnensumpf selektives Lösungsmittel, das noch mit Kohlenwasserstoffen verunreinigt ist, die bevorzugt in einem Verdampfer abgetrennt und erneut dem Kolonnensumpf zugeführt werden unter Erhalt eines gereinigten Lösungsmittels, das bevorzugt zumindest teilweise in den oberen Bereich der Kolonne recycliert wird.
Eine bevorzugte Verfahrensvariante ist dadurch gekennzeichnet, daß man aus der Kolonne, in der man die Verfahrensschritte (I) und (II) durchführt, aus einer Zone mit relativ erhöhter Konzentration an Acetylenen einen Strom abzieht und ihn erneut der Kolonne zuführt, bevorzugt in den obersten Bereich der katalytisch aktiven Zone derselben. Dadurch wird eine Erhöhung der Ausbeute an 1,3-Butadien erreicht.

Bevorzugt wird das Verfahren in der Weise durchgeführt, daß man mindestens eine Maßnahme trifft, um die Temperatur der Flüssigkeit im Sumpf der Kolonne abzusenken.
Nach dieser Verfahrensvariante wird die Temperaturbelastung des Reaktionsgemisches reduziert.

Bevorzugt senkt man die Temperatur der Flüssigkeit im Sumpf der Kolonne um 10 bis 80°C, insbesondere auf einen Wert im Bereich von 100 bis 170°C, bevorzugt von 140 bis 160°C, ab.

Eine bevorzugte Maßnahme zur Absenkung der Temperatur der Flüssigkeit im Sumpf der Kolonne ist erfindungsgemäß die Zuführung eines Zwischensiederstroms in den unteren Kolonnenbereich oder in den Sumpfverdampfer der Kolonne. Der Begriff Zwischensieder bezeichnet vorliegend einen Kohlenwasserstoff oder ein Gemisch von Kohlenwasserstoffen, der oder das über seinen Siedepunkt definiert wird:

Dieser muß vorliegend oberhalb des Siedepunkts von 1,3-Butadien und unterhalb des Siedepunkts des Lösungsmittels beziehungsweise Lösungsmittelgemisches liegen.

Bevorzugt führt man als Zwischensieder einen Stoff oder ein Stoffgemisch zu, der oder das bereits im Verfahren vorhanden ist.

Als Zwischensieder besonders geeignet ist ein Stoff oder ein Gemisch von Stoffen mit jeweils 5 Kohlenstoffatomen pro Molekül, bevorzugt ein oder mehrere Alkane und/oder ein oder mehrere Alkene.

Besonders bevorzugt führt man als Zwischensieder einen oder mehrere der Stoffe 2-Methyl-buten (2), 3-Methyl-buten (1), n-Pentan, i-Pentan, n-Penten (1) und n-Penten(2) zu.

Bevorzugt steht der Mengenstrom des Zwischensieders zum Mengenstrom des zugeführten C₄-Schnitts im Verhältnis von 0,001/1 bis 0,25/1, bevorzugt von 0,002/1 bis 0,15/1, besonders bevorzugt von 0,004/1 bis 0,008/1.

Insbesondere ist es auch möglich, als Zwischensiederstrom einen Sumpfstrom aus der Destillationskolonne zur Gewinnung von Rein-1,3-Butadien zuzuführen.

Eine zweite Maßnahme, die man erfindungsgemäß zur Absenkung der Temperatur der Flüssigkeit im Sumpf der Kolonne zusätzlich oder alternativ zur bereits dargelegten Zuführung eines Zwischensiederstroms treffen kann, besteht darin, daß man den Gehalt der niedriger siedenden Komponenten aus dem selektiven Lösungsmittel, insbesondere den Wasserdampfgehalt desselben im unteren Kolonnenbereich erhöht, indem man in den unteren Kolonnenbereich einen Strom der niedriger siedenden Komponente des selektiven Lösungsmittels, insbesondere Wasserdampf, zuführt und den aus der Kolonne abgezogenen Strom an selektivem Lösungsmittel vor dessen partieller oder vollständiger Rückführung in die Kolonne um den zugeführten Anteil an niedriger siedender Komponente, insbesondere Wasserdampf, abreichert.

Bevorzugt steht der relative Mengenstrom an niedriger siedender Komponente, insbesondere Wasserdampf, bezogen auf den Mengenstrom des der Kolonne zugeführten C₄-Schnitts in einem Verhältnis von 0,2/1 bis 1,6/1, bevorzugt von 1,2 zu 1.

In geeigneter Weise wird die niedriger siedende Komponente des selektiven Lösungsmittels, insbesondere Wasser, der Kolonne in Dampfform zugeführt, bevorzugt bei einem Druck gleich oder geringfügig oberhalb des Sumpfdrucks der Kolonne.

Ein im vorliegenden Verfahren besonders geeignetes selektives Lösungsmittel ist wie vorstehend ausgeführt N-Methylpyrrolidon, abgekürzt als NMP bezeichnet, bevorzugt in wässriger Lösung, insbesondere mit 8 bis 10 Gew.-% Wasser, besonders bevorzugt mit 8, 3 Gew.-% Wasser.

Vor der Recyclierung des Stroms des selektiven Lösungsmittels kann man denselben um den zugeführten Anteil an niedriger siedender Komponente, insbesondere Wasserdampf, abreichern. Hierdurch wird die Zusammensetzung des selektiven Lösungsmittels, die für seine Selektivität wesentlich ist, kaum oder nicht verändert.

Alternativ zu den vorstehend beschriebenen kann man als Maßnahme zur Absenkung der Temperatur im Sumpf der Kolonne in der Sumpfflüssigkeit der Kolonne einen erhöhten 1,3-Butadiengehalt, insbesondere von 0,5 bis 5 Gew.-% bezogen auf das Gesamtgewicht der Sumpfflüssigkeit, bevorzugt von 1 bis 3 Gew.-%, besonders bevorzugt von 1,8 Gew.-%, zulassen und die Sumpfflüssigkeit nach dem Abziehen aus der Kolonne in einer Strippkolonne an 1,3-Butadien abreichern, wobei man als Strippdampf bevorzugt das dampfförmige Kopfprodukt der Kolonne einsetzt.

Es ist auch möglich, die Strippkolonne mit einem zusätzlichen Sumpfverdampfer auszustatten.

Es ist jedoch auch möglich, anstatt in einer separaten Strippkolonne die Butadienabreicherung in einem zusätzlichen, im untersten Bereich der Kolonne angeordneten Teilbereich durchzuführen.

In einer Ausführungsvariante werden die Verfahrensschritte I und II in einer Trennwandkolonne durchgeführt.

Hierfür wird eine Vorrichtung eingesetzt mit
- einer Trennwandkolonne, in der eine Trennwand in Kolonnenlängsrichtung unter Ausbildung eines oberen gemeinsamen Kolonnenbereichs, eines unteren gemeinsamen Kolonnenbereichs, eines Zulaufteils und eines Entnahmeteils angeordnet ist,
- mit Zuführung des Zulaufgemisches im mittleren Bereich des Zulaufteils, Zuführung des Extraktionsmittels im oberen Bereich des Zulaufteils,
- mit Zuführung von Wasserstoff im unteren gemeinsamen Kolonnenbereich, Abtrennung von nicht umgesetztem Wasserstoff aus dem Brüdenstrom von kondensierbaren Leichtsiedern in einem Kondensator am Kopf der Trennwandkolonne und Rückführung über einen Verdichter in den unteren gemeinsamen Kolonnenbereich sowie
- mit flüssiger oder dampfförmiger Abführung des Roh-1,3-Butadienstromes aus dem Entnahmeteil der Trennwandkolonne an einer unterhalb der entsprechenden im Zulaufteil für die Zuführung gelegenen Stelle und
- Weiterleitung des Roh-1,3-Butadienstromes zur Reindestillation (Verfahrensschritt III).

Das Ausgangsgemisch, der C₄-Schnitt, wird vorab verdampft und dampfförmig in den mittleren Bereich des Zulaufteils der Trennwandkolonne zugeführt. Im oberen Bereich des Zulaufteils der Trennwandkolonne wird das Extraktionsmittel aufgegeben, wobei die Zugabestelle für das Extraktionsmittel so gewählt wird, daß sie soweit unterhalb des oberen Endes der Trennwand angeordnet ist, daß sichergestellt ist, daß kein

Extraktionsmittel in den oberen gemeinsamen Kolonnenbereich und in den oberen Bereich des Entnahmeteils gelangt.

Aus dem Brüdenstrom werden im Kondensator, der am Kopf der Trennwandkolonne angeordnet ist, die kondensierbaren Leichtsieder, insbesondere Butane, Butene sowie gegebenenfalls C₃-Kohlenwasserstoffe auskondensiert und bevorzugt teilweise als Rücklauf auf den Kopf der Trennwandkolonne aufgegeben und im übrigen als Leichtsiederstrom ausgeschleust. Der nicht zur Hydrierung verbrauchte Wasserstoff wird gasförmig über einen Verdichter im unteren gemeinsamen Kolonnenteil erneut zugeführt. Verbrauchter Wasserstoff wird als Frischwasserstoff ergänzt. Es ist jedoch auch möglich, alternativ oder zusätzlich zur Rückführung des nicht verbrauchten Wasserstoffs in den unteren gemeinsamen Kolonnenbereich denselben über den Sumpfverdampfer der Kolonne zurückzuführen. Die Einspeisung des Wasserstoffs in den Sumpfverdampfer bietet den Vorteil einer deutlichen Temperaturabsenkung des Sumpfprodukts und erlaubt eine bessere Abtrennung der Kohlenwasserstoffe aus dem Sumpfprodukt, ohne die für das Extraktionsmittel maximal zulässigen Betriebstemperaturen zu überschreiten.

Der Roh-1,3-Butadienstrom wird aus dem unteren Bereich des Entnahmeteils der Trennwandkolonne dampfförmig oder flüssig abgezogen, an einer Stelle, die unterhalb der entsprechenden im Zulaufteil für die Zuführung des C₄-Schnitts gelegenen Stelle angeordnet ist. Hierbei muß die Abführung soweit oberhalb des unteren Endes der Trennwand angeordnet sein, daß sichergestellt ist, daß kein Extraktionsmittel aus dem unteren gemeinsamen Kolonnenbereich in den Bereich des Entnahmeteils gelangen kann, der oberhalb der Abführung des 1,3-Butadien-haltigen Stromes liegt.

Sämtliche Kolonnenbereiche können mit üblichen destillativen Einbauten ausgestattet sein. Zusätzlich muß mindestens ein Bereich des Entnahmeteils mit reaktiven Einbauten ausgestattet sein, das heißt mit Einbauten, die die Selektivhydrierung heterogen katalysieren. Hierfür können, wie bereits dargestellt, übliche destillative Einbauten eingesetzt werden, auf die heterogene Katalysatoren aufgebracht wurden oder aber bevorzugt sogenannte TLC-Packungen. Zusätzlich zum oben definierten Teilbereich des Entnahmeteils kann auch der gesamte obere Teilbereich des Entnahmeteils mit reaktiven Einbauten ausgestattet sein.

In einer weiteren Ausführungsvariante wird eine Vorrichtung zur Durchführung des erfindungsgemäßen Verfahrens zur Verfügung gestellt, wonach die Trennwandkolonne durch thermisch gekoppelte Kolonnen ersetzt wird, bevorzugt mit jeweils eigenem Sumpfverdampfer und/oder Kondensator.

Diese Anordnungen sind hinsichtlich des Energiebedarfs mit einer Trennwandkolonne gleichwertig. Bei diesen Vorrichtungsvarianten ist es möglich, die beiden Kolonnen mit unterschiedlichen Drücken zu betreiben. Da der Wasserstoffpartialdruck für die Selektivhydrierung etwa 1 bis 10 bar beträgt, müssen die wasserstofführenden Anlagenteile für einen entsprechend höheren Druck ausgeführt sein. Durch Verwendung von thermisch gekoppelten Kolonnen, bei denen nur eine der Kolonnen auf den erhöhten Betriebsdruck ausgelegt werden muß, lassen sich die Investitionskosten verringern. Die Vorrichtungsvarianten mit thermisch gekoppelten Kolonnen bieten weiterhin dann Vorteile, wenn Katalysatoren mit niedriger Standzeit eingesetzt werden. Durch Anordnung des Katalysators in einer Seitenkolonne ist es möglich, diese in doppelter Ausführung bereitzustellen, so daß sich bei einer erforderlichen Katalysatorregenerierung, -wäsche oder einem Katalysatoraustausch Stillstandzeiten entweder ganz vermeiden oder zumindest wesentlich reduzieren lassen.

Trennwandkolonnen sind bei Neuanlagen aus Kostengründen zu bevorzugen, dagegen eignen sich thermisch gekoppelte Kolonnen insbesondere für die Umrüstung bereits vorhandener Destillationskolonnen.

Die Erfindung wird im folgenden anhand einer Zeichnung und von Ausführungsbeispielen näher erläutert. Es zeigen im einzelnen:
- Figur 1: die schematische Darstellung einer ersten erfindungsgemäßen Vorrichtung mit einer Trennwandkolonne, die
- Figuren 2a bis 2d: schematische Darstellungen von thermisch gekoppelten Kolonnen mit gemeinsamem Sumpfverdampfer und Kondensator und
- Figuren 3 a bis 3d: schematische Darstellungen von thermisch gekoppelten Kolonnen mit jeweils eigenen Sumpfverdatnpfern und Kondensatoren.
- Figur 4: die schematische Darstellung einer Verfahrensvariante mit Zuführung eines Zwischensiederstroms,
- Figur 5: die schematische Darstellung einer Verfahrensvariante mit Zuführung von Wasserdampf und
- Figuren 6 und 7: schematische Darstellungen von zwei unterschiedlichen Verfahrensvarianten mit Festlegung eines höheren 1,3-Butadiengehalts im Kolonnensumpf.

In den Figuren werden gleiche Bezugszeichen für gleiche oder entsprechende Ströme verwendet.

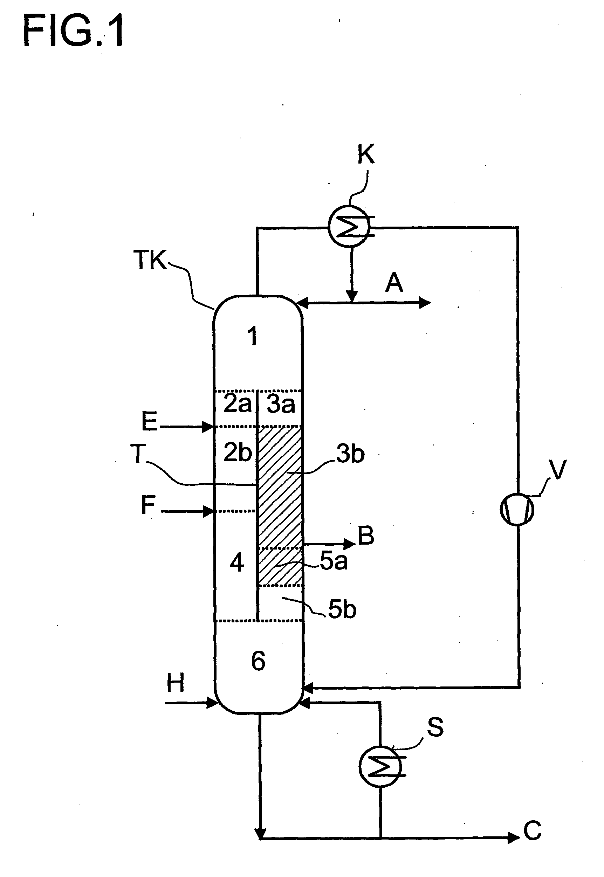

Die in Fig. 1 schematisch dargestellte Ausführungsvariante zeigt eine Trennwandkolonne TK mit einer in Kolonnenlängsrichtung angeordneten Trennwand T, die die Trennwandkolonne TK in einen oberen gemeinsamen Kolonnenbereich 1, einen unteren gemeinsamen Kolonnenbereich 6, einen Zulaufteil 2a, 2b, 4 und einen Entnahmeteil 3a, 3b, 5a, 5b aufteilt. Der C₄-Schnitt wird über Zuführung F zwischen die Teilbereiche 2b und 4 des Zulaufteils zugeführt, das Extraktionsmittel E zwischen die Teilbereiche 2a und 2b

des Zulaufteils und Wasserstoff H in den unteren gemeinsamen Kolonnenbereich 6. Aus dem Brüdenstrom werden im Kondensator K die kondensierbaren Leichtsieder abgetrennt, teilweise als Rücklauf auf den Kopf der Kolonne gegeben und im übrigen als Leichtsiederstrom A ausgeschleust. Der gasförmige Wasserstoff wird über den Verdichter V der Trennwandkolonne TK in deren unteren gemeinsamen Kolonnenbereiche 6 erneut zugeführt. Die Kolonne verfügt über einen Sumpfverdampfer S, über den das Sumpfprodukt teilweise erneut in den unteren gemeinsamen Kolonnenbereich 6 zugeführt wird, wobei ein Teil des Sumpfprodukts, ohne Rückführung über den Sumpfverdampfer, als Schwersiederstrom C aus der Trennwandkolonne ausgeschleust wird.

Der Zulaufteil der Trennwandkolonne TK ist aus den Teilbereichen 2a, 2b und 4 gebildet, wobei der Teilbereich 2a oberhalb der Zuführung des Extraktionsmittels E liegt, der Teilbereich 2b zwischen der Zuführung von Extraktionsmittel E und C₄-Schnitt F und der Teilbereich 4 unterhalb der Zuführung des C₄-Schnitts F angeordnet ist. Der Entnahmeteil der Trennwandkolonne wird aus den Teilbereichen 3a, 3b, 5a und 5b gebildet. Der Teilbereich 5b ist so dimensioniert, daß Extraktionsmittel aus dem unteren gemeinsamen Kolonnenbereich 6 nicht in den mit reaktiven Einbauten ausgestatteten Teilbereich 5a des Entnahmeteils gelangen kann. Der 1,3-Butadien-haltige Strom B wird dem Entnahmeteil der Trennwandkolonne TK zwischen den Teilbereichen 3b und 5a entnommen.

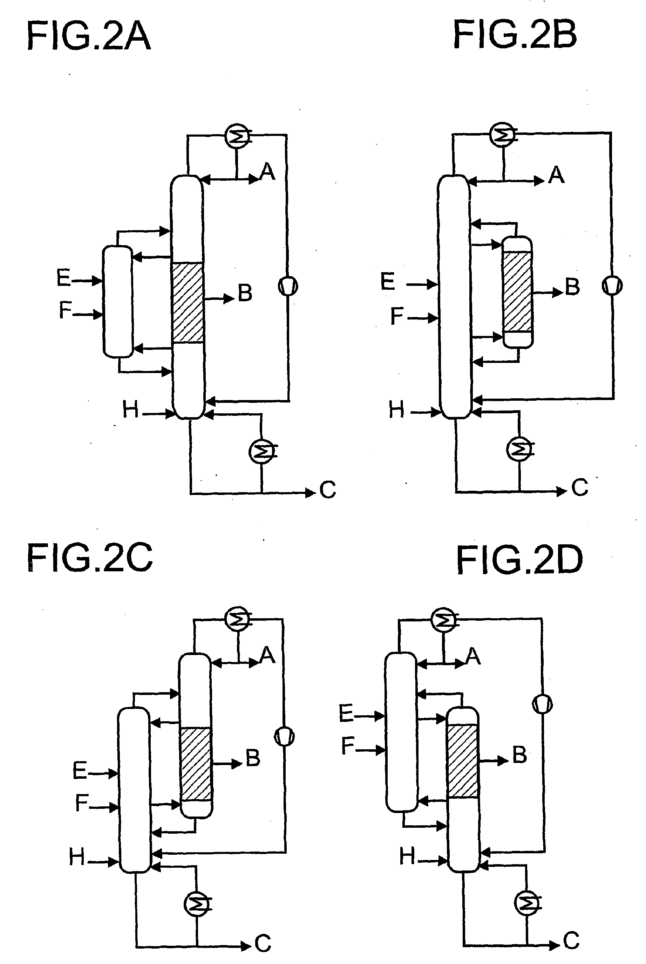

Die Fig. 2a bis 2d zeigen schematisch unterschiedliche Ausführungsformen und Vorrichtungsvarianten mit thermisch gekoppelten Destillationskolonnen mit jeweils gemeinsamem Sumpf und gemeinsamem Kondensator. Hierbei sind die Kolonnenbereiche 1, 2a, 2b, 3a, 3b, 4, 5a, 5b und 6 der Trennwandkolonne TK aus Fig. 1 in unterschiedlicher Weise auf jeweils zwei einzelnen Kolonnen aufgeteilt.

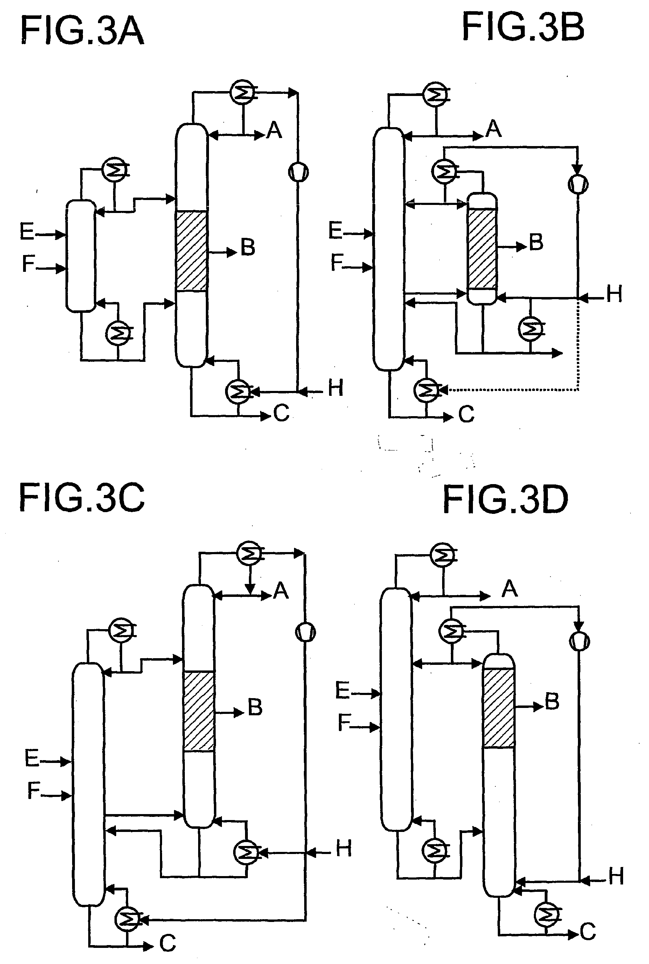

Die Fig. 3a bis 3d zeigen weitere Ausführungsvarianten von thermisch gekoppelten Kolonnen, wobei jede Kolonne einen eigenen Sumpfverdampfer und einen eigenen Kondensator aufweist. Der Rücklauf für jede einzelne Kolonne wird durch Kondensation in einem eigenen Kondensator erzeugt. Zur Verringerung des Energiebedarfs sind die Kondensatoren bevorzugt als Partialkondensatoren ausgeführt.

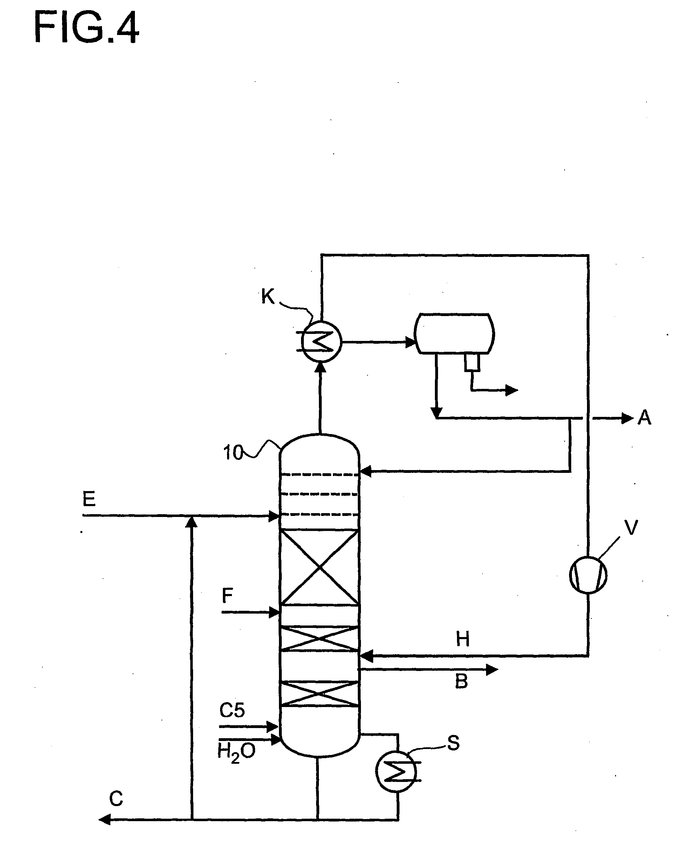

Figur 4 zeigt schematisch eine Anlage zur Durchführung der Ausführungsvariante ohne Trennwand mit Zuführung eines Zwischensiederstroms:
Einer einzigen Kolonne 10 wird das selektive Lösungsmittel E in deren oberen Bereich zugeführt, der C₄-Schnitt F in ihren mittleren Bereich und ein Wasserstoffstrom H unterhalb der Zuführung des Stromes F. Ein Roh-1,3-Butadienstrom B wird als Seitenstrom abgezogen. Die Kolonne ist im Bereich oberhalb der Zuführung des Stromes E mit Böden und unterhalb derselben mit Füllkörpern oder geordneten Packungen, die zumindest zum Teil katalytisch aktiv sein müssen, bestückt. Der Brüdenstrom wird kondensiert und als Leichtsiederstrom A, der überwiegend Butane und Butene enthält, abgezogen. Aus dem Kolonnensumpf wird Lösungsmittel in den Sumpfverdampfer S abgezogen, teilweise gereinigt und anschließend in den Strom E recycliert. Dem Sumpfverdampfer S wird ein Zwischensiederstrom C₅ zugeführt.

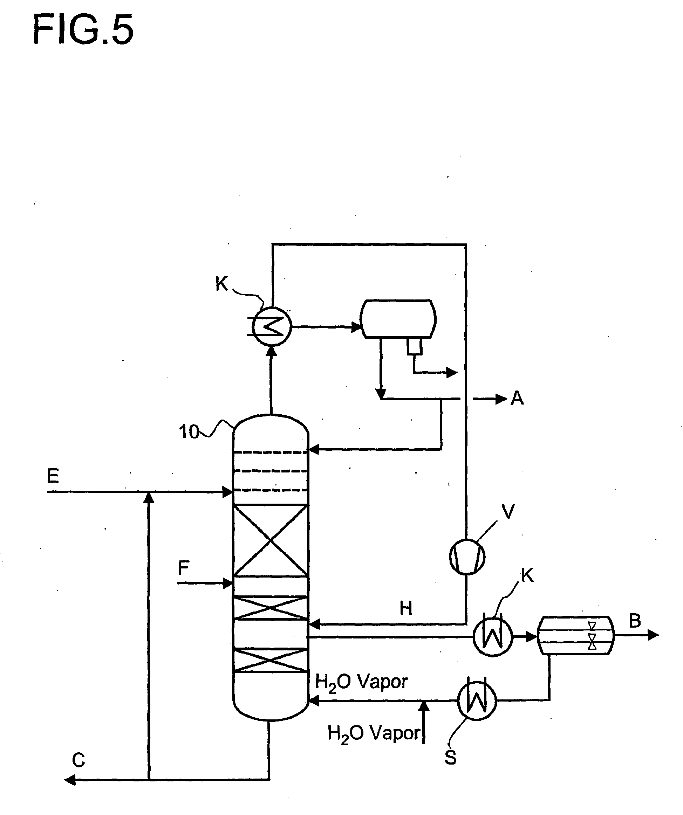

Figur 5 zeigt die schematische Darstellung einer Anlage zur Durchführung der bevorzugten Verfahrensvariante mit Zuführung eines Wasserstromes, bevorzugt dampfförmig (H₂O-vap) in deren unteren Bereich. Diese Wassermenge wird mittels einer Kondensation des Roh-1,3-Butadienstroms und einer Abtrennung der wässrigen Phase in einem Phasenscheider, bevorzugt deren Verdampfung, in die Kolonne im Kreis zurückgeführt.

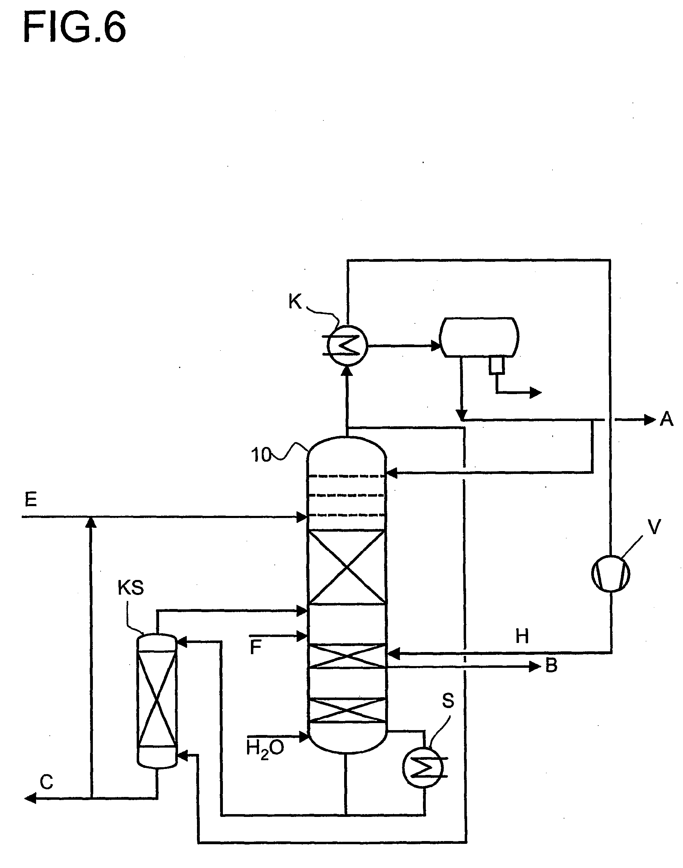

In der in Figur 6 dargestellten Alternative, die ein Verfahren mit Zulassung eines erhöhten 1,3-Butadiengehalts in der Sumpfflüssigkeit betrifft, wird die Sumpfflüssigkeit in einer getrennten Strippkolonne KS ausgestrippt. Hierfür kann, wie beispielhaft dargestellt, der Brüdenstrom der Kolonne 10 eingesetzt werden.

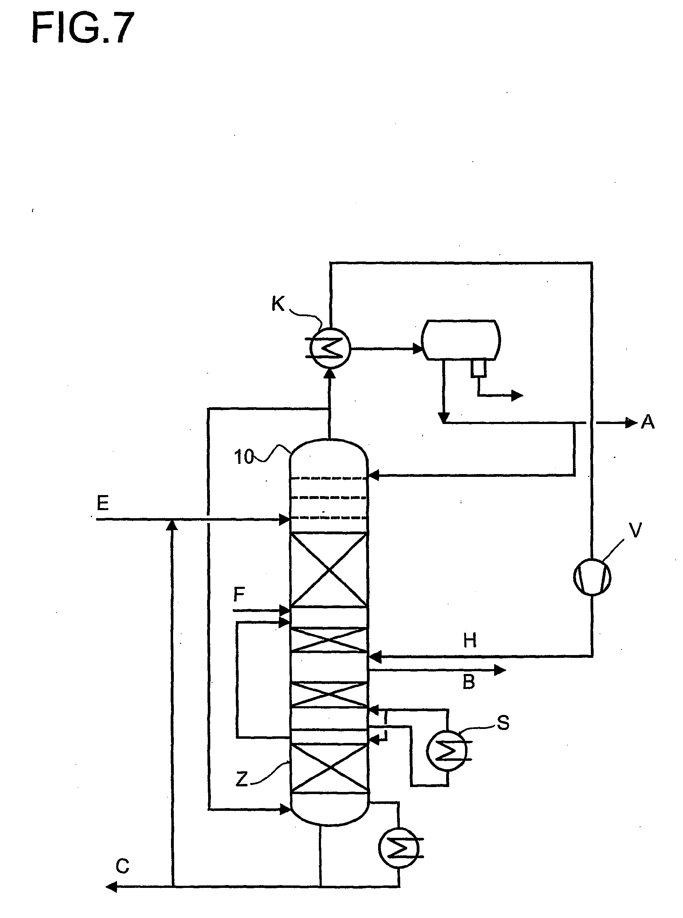

Figur 7 zeigt die schematische Darstellung einer weiteren Anlage zur Durchführung einer Verfahrensvariante mit erhöhter 1,3-Butadienkonzentration. In dieser Variante ist die Strippkolonne KS als zusätzlicher unterster Teil Z der Kolonne 10 angesetzt und von dieser gas- und flüssigkeitsdicht getrennt.

### Beispiele:

Einer Kolonne mit insgesamt 70 theoretischen Trennstufen, mit Kopfdruck von 4,5 bar, wurde auf der 45.Trennstufe, bei Zählung der Trennstufen von unten, ein Mengenstrom von kg/h eines C₄-Schnitts mit der eingangs bereits angegebenen Zusammensetzung zugeführt. Auf der Stufe 65 wurde als selektives Lösungsmittel eine wässrige, 8,3 Gew.-%

### Beispiele:

Einer Kolonne mit insgesamt 70 theoretischen Trennstufen, mit Kopfdruck von 4,5 bar, wurde auf der 45.Trennstufe, bei Zählung der Trennstufen von unten, ein Mengenstrom von 1,5 kg/h eines C₄-Schnitts mit der eingangs bereits angegebenen Zusammensetzung zugeführt. Auf der Stufe 65 wurde als selektives Lösungsmittel eine wässrige, 8,3 Gew.-% NMP-haltige Lösung zugeführt. Ein Wasserstoffstrom von 15 g/h wurde auf Stufe 11. zugeführt. Von Stufe 10 wurde ein Roh-1,3-Butadienstrom abgezogen. Im Kolonnensumpf stellte sich eine Temperatur von 186°C ein.

Unter den oben genannten Versuchsbedingungen wurden in den Kolonnensumpf jeweils die nachfolgend aufgeführten Mengenströme des Zwischensieders 2-Methyl-buten zugeführt.

Die jeweils erreichte Absenkung der Temperatur der Sumpfflüssigkeit ist der nachfolgenden Tabelle zu entnehmen:

| Beispiel Nr. | C₅-Mengenstrom (g/h) | Temperatur Sumpfflüssigkeit (°C) |
|---|---|---|
| 1 | 3 | 184,6 |
| 2 | 6 | 183,4 |
| 3 | 30 | 175,2 |
| 4 | 60 | 168,1 |
| 5 | 120 | 159,2 |
| 6 | 240 | 150,2 |

### Beispiel 7:

Unter denselben Versuchsbedingungen wie eingangs beschrieben wurde anstelle eines C₅-Stroms ein Wasserdampfstrom von 300 g/h in den unteren Bereich der Kolonne zugeführt.

### Beispiel 8:

Es wurde analog wie zu Beispiel 7 beschrieben vorgegangen, jedoch eine höhere Dampfmenge, von 2010 g/h, zugeführt. Dadurch wurde eine Temperaturabsenkung in der Sumpfflüssigkeit auf 165 °C erreicht.

## Patentansprüche

1. Verfahren zur Aufarbeitung eines C₄-Schnittes umfassend die Verfahrensschritte
- Extraktivdestillation (I),
- Selektivhydrierung acetylenischer Verunreinigungen an einem heterogenen Katalysator (II), wobei nach den Verfahrensschritten I und II ein Roh-1,3-Butadienstrom, enthaltend das Wertprodukt 1,3-Butadien in einem Anteil von mindestens 80 Gew.-%, erhalten wird und
- Destillation des Roh-1,3-Butadienstromes zur Gewinnung von Rein-1,3-Butadien (III), enthaltend das Wertprodukt 1,3-Butadien in einem Anteil von mindestens 99 Gew.-%,
**dadurch gekennzeichnet, daß** die Verfahrensschritte I und II in einer einzigen Kolonne und der Verfahrensschritt III in einer zweiten Kolonne durchgeführt wird, wobei der heterogene Katalysator für die Selektivhydrierung acetylenischer Verunreinigungen in Form von reaktiven Einbauten vorliegt.

2. Verfahren zur Aufarbeitung eines C₄-Schnittes umfassend die Verfahrensschritte
- Extraktivdestillation (I),
Selektivhydrierung acetylenischer Verunreinigungen an einem heterogenen Katalysator (II), wobei nach den Verfahrensschritten I und II ein Roh-1,3-Butadienstrom, enthaltend das Wertprodukt 1,3-Butadien in einem Anteil von mindestens 80 Gew.-%, erhalten wird, und
Destillation des Roh-1,3-Butadienstromes zur Gewinnung von Rein-1,3-Butadien (III), enthaltend das Wertprodukt 1,3-Butadien in einem Anteil von mindestens 99 Gew.-%,
**dadurch gekennzeichnet, daß** die Verfahrensschritte I und II in thermisch gekoppelten Kolonnen, d.h. Anordnungen von mindestens zwei Kolonnen, wobei jede der Kolonnen mit jeder anderen mindestens zwei Verknüpfungen an räumlich getrennten Stellen aufweist, und der Verfahrensschritt III in einer weiteren Kolonne durchgeführt wird, wobei der heterogene Katalysator für die Selektivhydrierung acetylenischer Verunreinigungen in Form von reaktiven Einbauten vorliegt.

3. Verfahren nach Anspruch 1 oder 2, **dadurch gekennzeichnet, dass** die reaktiven Einbauten übliche destillative Einbauten sind, auf die der heterogene Katalysator für die Selektivhydrierung acetylenischer Verunreinigungen aufgebracht ist, insbesondere Kolonnenböden, Formkörper oder Packungen oder in Form von Drahtnetzen mit Taschen vorliegen, in die der heterogene Katalysator für die Selektivhydrierung acetylenischer Verunreinigungen eingebettet ist.

4. Verfahren nach einem der Ansprüche 1 bis 3, **dadurch gekennzeichnet, daß** als Extraktionsmittel für den Verfahrensschritt I N-Methylpyrrolidon, bevorzugt in wäßriger Lösung, insbesondere mit 8 bis 10 Gew.-% Wasser, besonders bevorzugt mit 8,3 Gew.-% Wasser, eingesetzt wird.

5. Verfahren nach einem der Ansprüche 1 bis 4, **dadurch gekennzeichnet, daß** als heterogener Katalysator für die Selektivhydrierung (Verfahrensschritt II) eine TLC-Packung eingesetzt wird.

6. Verfahren nach einem der Ansprüche 1 bis 5, **dadurch gekennzeichnet, daß** man aus der Kolonne, in der man die Verfahrensschritte I und II durchführt, aus einer Zone mit relativ erhöhter Konzentration an Acetylenen einen Strom abzieht und ihn erneut der Kolonne zuführt, bevorzugt in den obersten Bereich der katalytisch aktiven Zone derselben.

7. Verfahren nach einem der Ansprüche 1 bis 6, **dadurch gekennzeichnet, daß** man mindestens eine Maßnahme trifft, um die Temperatur der Flüssigkeit im Sumpf der Kolonne abzusenken.

8. Verfahren nach Anspruch 7, **dadurch gekennzeichnet, daß** man die Temperatur der Flüssigkeit im Sumpf der Kolonne um 10 bis 80°C, insbesondere auf einen Wert im Bereich von 100 bis 170°C, bevorzugt von 140 bis 160°C, absenkt.

9. Verfahren nach Anspruch 7 oder 8, **dadurch gekennzeichnet, daß** man in den unteren Kolonnenbereich oder in den Sumpfverdampfer der Kolonne einen Zwischensiederstrom zuführt.

10. Verfahren nach Anspruch 9, **dadurch gekennzeichnet, daß** man als Zwischensieder einen Stoff oder ein Stoffgemisch zuführt, der oder das bereits im Verfahren vorhanden ist.

11. Verfahren nach Anspruch 9 oder 10, **dadurch gekennzeichnet, daß** man als Zwischensieder einen Stoff oder ein Gemisch von Stoffen mit jeweils 5 Kohlenstoffatomen pro Molekül, bevorzugt ein oder mehrere Alkane und/oder ein oder mehrere Alkene, zuführt.

12. Verfahren nach Anspruch 11, **dadurch** gekennzeichent, daß man als Zwischensieder einen oder mehrere der Stoffe: 2-Methyl-buten (2), 3-Methyl-buten (1), n-Pentan, i-Pentan, n-Penten (I) und n-Penten (2) zuführt.

13. Verfahren nach einem der Ansprüche 9 bis 12, **dadurch gekennzeichnet, daß** der Mengenstrom des Zwischensieders zum Mengenstrom des zugeführten C₄-Schnitts im Verhältnis von 0,001/1 bis 0,25/1, bevorzugt 0,002/1 bis 0,15/l, besonders bevorzugt 0,004/l bis 0,008/l steht.

14. Verfahren nach einem der Ansprüche 7 oder 8, **dadurch gekennzeichnet, daß** man den Gehalt der niedriger siedenden Komponenten aus dem selektiven Lösungsmittel, insbesondere den Wasserdampfgehalt desselben im unteren Kolonnenbereich erhöht, indem man in den unteren Kolonnenbereich einen Strom der niedriger siedenden Komponente des selektiven Lösungsmittels, insbesondere Wasserdampf, zuführt und den aus der Kolonne abgezogenen Strom an selektivem Lösungsmittel vor dessen partieller oder vollständiger Rückführung in die Kolonne um den zugeführten Anteil an niedriger siedender Komponente, insbesondere Wasserdampf, abreichert.

15. Verfahren nach Anspruch 14, **dadurch gekennzeichnet, daß** der relative Mengenstrom an niedriger siedender Komponente, insbesondere Wasserdampf, bezogen auf den Mengenstrom des der Kolonne zugeführten C₄-Schnitts in einem Verhältnis von 0,2/l bis 1,6/1, bevorzugt von 1,2 zu 1, steht.

16. Verfahren nach Anspruch 14 oder 15, **dadurch gekennzeichnet, daß** die niedriger siedende Komponente des selektiven Lösungsmittels, insbesondere Wasser, der Kolonne in Dampfform zugeführt wird, bei einem Druck gleich oder geringfügig oberhalb dem Sumpfdruck der Kolonne.

17. Verfahren nach Anspruch 7 oder 8, **dadurch gekennzeichnet, daß** man in der Sumpfflüssigkeit der Kolonne einen erhöhten 1,3-Butadiengehalt, insbesondere von 0,5 bis 5 Gew.-% bezogen auf das Gesamtgewicht der Sumpfflüssigkeit, bevorzugt von 1 bis 3 Gew.-%, besonders bevorzugt von 1,8 Gew.-% zuläßt und die Sumpfflüssigkeit nach dem Abziehen aus der Kolonne in einer Strippkolonne an 1,3-Butadien abreichert, wobei man als Strippdampf bevorzugt das dampfförmige Kopfprodukt der Kolonne einsetzt.

18. Verfahren nach einem der Ansprüche 1, 3, 4, 5 oder 6, **dadurch gekennzeichnet, daß** die Verfahrensschritte I und II in einer Trennwandkolonne durchgeführt werden.

19. Verfahren nach Anspruch 18, **dadurch gekennzeichnet, dass** der Verfahrensschritt I in einer Trennwandkolonne (TK) durchgeführt wird,
- in der eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2a, 2b, 4) mit von oben nach unten angeordneten Teilbereichen (2a, 2b, 4) sowie eines Entnahmeteils (3a, 3b, 5a, 5b) angeordnet ist, wobei der Teilbereich (5a) des Entnahmeteils (3a, 3b, 5a, 5b) reaktive Einbauten aufweist,
- mit Zuführung des C₄-Schnitts (F) im mittleren Bereich des Zulaufteils (2a, 2b, 4), zwischen den Teilbereichen (2b und 4) desselben, Zuführung von Extraktionsmittel (E) im oberen Bereich des Zulaufteils (2a, 2b, 4), zwischen den Teilbereichen (2a und 2b),
- mit Zuführung von Wasserstoff (H) unterhalb des Teilbereichs (5a), Abtrennung von nichtumgesetztem Wasserstoff aus dem Brüdenstrom der Trennwandkolonne (TK) von kondensierbaren Leichtsiedern im Kondensator (K) und Rückführung über einen Verdichter (V) in den unteren gemeinsamen Kolonnenbereich (6) sowie
- mit Abführung des 1,3-Butadien-haltigen Stromes (B) aus dem Entnahmeteil (3a, 3b, 5a, 5b), der Trennwandkolonne (TK) an einer Stelle zwischen den Teilbereichen (3b) und (5a) und dass
- der Strom (B) zur Reindestillation (Verfahrensschritt III) weitergeleitet wird.

20. Verfahren nach Anspruch 19, **dadurch gekennzeichnet, daß** die aus dem Brüdenstrom im Kondensator (K) auskondensierten Leichtsieder teilweise als Rücklauf auf den Kopf der Trennwandkolonne (TK) aufgegeben und im übrigen als Leichtsiederstrom (A) ausgeschleust werden.

21. Vorrichtung zur Durchführung des Verfahrens nach Anspruch 19 oder 20, **dadurch gekennzeichnet, daß** die Trennwandkolonne (TK) reaktive Einbauten im Teilbereich (5a) des Entnahmeteils (3a, 3b, 5a, 5b) aufweist.

22. Vorrichtung nach Anspruch 21, **dadurch gekennzeichnet, daß** reaktive Einbauten zusätzlich in den oberen Teilbereichen (3a, 3b) des Entnahmeteils (3a, 3b, 5a, 5b), bevorzugt im Teilbereich (3b), besonders bevorzugt in den Teilbereichen (3a und 3b), enthalten sind.

## Claims

1. A process for the work-up of a C₄ fraction, comprising the process steps
- Extractive distillation (I),
- Selective hydrogenation of acetylenic impurities over a heterogeneous catalyst (II), a crude 1,3-butadiene stream comprising the target product 1,3-butadiene in a fraction of at least 80% by weight being obtained following process steps I and II and
- Distillation of the crude 1,3-butadiene stream for isolating pure 1,3-butadiene (III) comprising the target product 1,3-butadiene in a fraction of at least 99% by weight,
wherein the process steps I and II are carried out in a single column and the process step III is carried out in a second column, the heterogeneous catalyst used for the selective hydrogenation of acetylenic impurities being present in the form of reactive internals.

2. A process for the work-up of a C₄ fraction comprising the process steps
- Extractive distillation (I),
- Selective hydrogenation of acetylenic impurities over a heterogeneous catalyst (II), a crude 1,3-butadiene stream comprising the target product 1,3-butadiene in a fraction of at least 80% by weight being obtained following process steps I and II and
- Distillation of the crude 1,3-butadiene stream for isolating pure 1,3-butadiene (III) comprising the target product 1,3-butadiene in a fraction of at least 99% by weight,
wherein the process steps I and II are carried out in thermally coupled columns i.e. arrangements of at least two columns, each column having at least two links to each other column, at spatially separate points, and the process step III is carried out in a further column, the heterogeneous catalyst used for the selective hydrogenation of acetylenic impurities being present in the form of reactive internals.

3. The process according to claim 1 or 2, wherein the reactive internals are customary distillation internals, to which the heterogeneous catalyst for the selective hydrogenation of acetylenic impurities is applied, in particular column trays, shaped bodies or packings or are present in the form of wire mesh with pockets in which the heterogeneous catalyst for the selective hydrogenation of acetylenic impurities is embedded.

4. The process according to any of claims 1 to 3, wherein the extractant used for the process step I is N-methylpyrrolidone, preferably in aqueous solution, in particular with from 8 to 10% by weight of water, particularly preferably with 8.3% by weight of water.

5. The process according to any of claims 1 to 4, wherein the heterogeneous catalyst used for the selective hydrogenation (process step II) is a TLC packing.

6. The process according to any of claims 1 to 5, wherein a stream is taken off from the column in which process steps I and II are conducted from a zone having a relatively high concentration of acetylenes and this stream is supplied again to the column, preferably to the topmost region of its catalytically active zone.

7. The process according to any of claims 1 to 6, wherein at least one measure is taken to lower the temperature of the liquid in the bottom of the column.

8. The process according to claim 7, wherein the temperature of the liquid in the bottom of the column is lowered by from 10 to 80°C, in particular to a level in the range from 100 to 170°C, preferably from 140 to 160°C.

9. The process according to claim 7 or 8, wherein a middle boiler stream is supplied to the lower region of the column or to the bottoms vaporizer of the column.

10. The process according to claim 9, wherein as middle boiler a substance or mixture of substances is supplied which is already present in the process.

11. The process according to claim 9 or 10, wherein as middle boiler a substance or a mixture of substances having in each case 5 carbon atoms per molecule is supplied, preferably one or more alkanes and/or more alkenes.

12. The process according to claim 11, wherein as middle boiler one or more of the following substances is supplied: 2-methyl-2-butene, 3-methyl-1-butene, n-pentane, isopentane, n-pent-1-ene and n-pent-2-ene.

13. The process according to any of claims 9 to 12, wherein the ratio of the volume flow of the middle boiler to the volume flow of the C₄ fraction supplied is from 0.001/1 to 0.25/1, preferably from 0.002/1 to 0.15/1, with particular preference from 0.004/1 to 0.008/1.

14. The process according to either of claims 7 and 8, wherein the amount of the relatively low-boiling components from the selective solvent, in particular its steam content, is increased in the lower region of the column by supplying a stream of the relatively low-boiling component of the selective solvent, especially steam, to the lower region of the column and depleting the stream of selective solvent taken off from the column, prior to its partial or complete recycling to the column, by the supplied fraction of relatively low-boiling component, especially steam.

15. The process according to claim 14, wherein the ratio of the relative volume flow of relatively low-boiling component, especially steam, to the volume flow of the C₄ fraction supplied to the column is.from 0.2/1 to 1.6/1, preferably 1.2:1.

16. The process according to claim 14 or 15, wherein the relatively low-boiling component of the selective solvent, especially water, is supplied to the column in vapor form, at a pressure equal to or slightly above the bottom pressure of the column.

17. The process according to claim 7 or 8, wherein in the bottoms liquid of the column an increased 1,3-butadiene content, in particular from 0.5 to 5% by weight based on the total weight of the bottoms liquid, preferably from 1 to 3% by weight, with particular preference 1.8% by weight is allowed and the bottoms liquid is depleted, after it has been taken off from the column, of 1,3-butadiene in a stripping column, using as stripping vapor preferably the vaporous top product of the column.

18. The process according to any of claims 1, 3, 4, 5 and 6, wherein the process steps I and II are conducted in a dividing wall column.

19. The process according to claim 18, wherein process step I is carried out in a dividing wall column (TK)
- in which a dividing wall (T) is arranged in the longitudinal direction of the column to form an upper common column region (1), a lower common column region (6), an inflow section (2a, 2b, 4) having subsections (2a, 2b, 4) arranged from top to bottom, and an offtake section (3a, 3b, 5a, 5b), the subsection (5a) of the offtake section (3a, 3b, 5a, 5b) having reactive internals,
- with introduction of the C₄ fraction (F) in the middle region of the inflow section (2a, 2b, 4), between the subsections (2b and 4) of the latter, introduction of extractant (E) in the upper region of the inflow section (2a, 2b, 4) between the subsections (2a and 2b),
- with introduction of hydrogen (H) below the subsection (5a), separation of unreacted hydrogen in the vapor stream of the dividing wall column (TK) from condensable low boilers in a condenser (K) and recirculation via a compressor (V) to the lower common column region (6) and
- with discharge of the 1,3-butadiene-containing stream (B) from the offtake section (3a, 3b, 5a, 5b) of the dividing wall column (TK) at a point between the subsections (3b) and (5a), and wherein
- the stream (B) is further transported to the final distillation (process step III).

20. The process according to claim 19, wherein the low boilers condensed from the vapor stream in the condenser (K) are partly returned as runback to the top of the dividing wall column (TK) and otherwise discharged as low boiler stream (A).

21. An apparatus for carrying out the process according to claim 19 or 20, wherein the dividing wall column (TK) has reactive internals in the subsection (5a) of the offtake section (3a, 3b, 5a, 5b).

22. The apparatus according to claim 21, wherein reactive internals are present additionally in the upper subsections (3a, 3b) of the offtake section (3a, 3b, 5a, 5b), preferably in the subsection (3b), particularly preferably in the subsections (3a and 3b).

## Revendications

1. Procédé de régénération d'une coupe en C₄ comprenant les étapes de procédé suivantes :
- distillation extractive (I),
- hydrogénation sélective d'impuretés acétyléniques sur un catalyseur hétérogène (II), un flux de 1,3-butadiène brut, contenant le produit de valeur 1,3-butadiène dans une proportion d'au moins 80 % en poids, étant obtenu après les étapes de procédé I et II, et
- distillation du flux de 1,3-butadiène brut en vue de l'obtention de 1,3-butadiène pur (III), contenant le produit de valeur 1,3-butadiène dans une proportion d'au moins 99 % en poids,
**caractérisé en ce que** les étapes de procédé I et II sont effectuées dans une colonne unique et **en ce que** l'étape de procédé III est effectuée dans une deuxième colonne, le catalyseur hétérogène pour l'hydrogénation sélective d'impuretés acétyléniques étant présent sous la forme de garnissages réactifs.

2. Procédé de régénération d'une coupe en C₄ comprenant les étapes de procédé suivantes :
- distillation extractive (I),
- hydrogénation sélective d'impuretés acétyléniques sur un catalyseur hétérogène (II), un flux de 1,3-butadiène brut, contenant le produit de valeur 1,3-butadiène dans une proportion d'au moins 80 % en poids, étant obtenu après les étapes de procédé I et II, et
- distillation du flux de 1,3-butadiène brut en vue de l'obtention de 1,3-butadiène pur (III), contenant le produit de valeur 1,3-butadiène dans une proportion d'au moins 99 % en poids,
**caractérisé en ce que** les étapes de procédé I et II sont effectuées dans des colonnes thermiquement couplées, c'est-à-dire des agencements d'au moins deux colonnes, chacune des colonnes présentant avec chaque autre au moins deux connexions en des points séparés spatialement, et **en ce que** l'étape de procédé III est effectuée dans une autre colonne, le catalyseur hétérogène pour l'hydrogénation sélective d'impuretés acétyléniques étant présent sous la forme de garnissages réactifs.

3. Procédé selon la revendication 1 ou 2, **caractérisé en ce que** les garnissages réactifs sont des garnissages de distillation usuels sur lesquels le catalyseur hétérogène pour l'hydrogénation sélective d'impuretés acétyléniques est appliqué, en particulier des plateaux de colonnes, des corps moulés ou des conditionnements ou sous forme de filets métalliques avec des poches, dans lesquels le catalyseur hétérogène pour l'hydrogénation sélective d'impuretés acétyléniques est incorporé.

4. Procédé selon l'une quelconque des revendications 1 à 3, **caractérisé en ce que** de la N-méthylpyrrolidone, de manière préférée en solution aqueuse, en particulier avec 8 à 10 % en poids d'eau, de manière particulièrement préférée avec 8,3 % en poids d'eau, est utilisée en tant qu'agent d'extraction pour l'étape de procédé I.

5. Procédé selon l'une quelconque des revendications 1 à 4, **caractérisé en ce qu'**un conditionnement TLC est utilisé en tant que catalyseur hétérogène pour l'hydrogénation sélective (étape de procédé II).

6. Procédé selon l'une quelconque des revendications 1 à 5, **caractérisé en ce qu'**un flux en provenance d'une zone à concentration relativement élevée en acétylènes est extrait de la colonne dans laquelle on effectue les étapes de procédé I et II, et **en ce qu'**il est amené de manière renouvelée à la colonne, de manière préférée dans le secteur supérieur de la zone catalytiquement active de cette même colonne.

7. Procédé selon l'une quelconque des revendications 1 à 6, **caractérisé en ce qu'**au moins une mesure est prise pour faire baisser la température du liquide dans le pied de la colonne.

8. Procédé selon la revendication 7, **caractérisé en ce que** la température du liquide dans le pied de la colonne est abaissée de 10 à 80°C, en particulier à une valeur dans la plage de 100 à 170°C, de manière préférée de 140 à 160°C.

9. Procédé selon la revendication 7 ou 8, **caractérisé en ce qu'**un flux de fraction intermédiaire est amené dans le secteur inférieur de colonne ou dans l'évaporateur de pied de la colonne.

10. Procédé selon la revendication 9, **caractérisé en ce qu'**une substance ou un mélange de substances déjà présent dans le procédé est amené en tant que fraction intermédiaire.

11. Procédé selon la revendication 9 ou 10, **caractérisé en ce qu'**une substance ou un mélange de substances ayant respectivement 5 atomes de carbone par molécule, de manière préférée un ou plusieurs alcanes et/ou un ou plusieurs alcènes, est amené en tant que fraction intermédiaire.

12. Procédé selon la revendication 11, **caractérisé en ce qu'**une ou plusieurs des substances suivantes : 2-méthylbutène (2), 3-méthylbutène (1), n-pentane, i-pentane, n-pentène (I) et n-pentène (2), est amenée en tant que fraction intermédiaire.

13. Procédé selon l'une quelconque des revendications 9 à 12, **caractérisé en ce que** le débit de matière de la fraction intermédiaire sur le débit de matière de la coupe en C₄ amenée se situe dans le rapport de 0,001/1 à 0,25/1, de manière préférée 0,002/1 à 0,15/1, de manière particulièrement préférée 0,004/1 à 0,008/1.

14. Procédé selon l'une quelconque des revendications 7 ou 8, **caractérisé en ce que** la teneur des composants à point d'ébullition plus faible issus du solvant sélectif, en particulier la teneur en vapeur d'eau de ce même solvant dans le secteur inférieur de colonne, est augmentée en amenant dans le secteur inférieur de colonne un flux des composants à point d'ébullition plus faible du solvant sélectif, en particulier de vapeur d'eau et en appauvrissant de la part en composants à point d'ébullition plus faible amenée, en particulier en vapeur d'eau, le flux de solvant sélectif extrait de la colonne avant son recyclage partiel ou complet dans la colonne.

15. Procédé selon la revendication 14, **caractérisé en ce que** le débit de matière relatif en composants à point d'ébullition plus faible, en particulier en vapeur d'eau, par rapport au débit de matière de la coupe en C₄ amenée à la colonne, se situe dans un rapport de 0,2/1 à 1,6/1, de manière préférée de 1,2 sur 1.

16. Procédé selon la revendication 14 ou 15, **caractérisé en ce que** les composants à point d'ébullition plus faible du solvant sélectif, en particulier l'eau, sont amenés à la colonne sous forme de vapeur, à une pression égale ou quelque peu supérieure à la pression de pied de la colonne.

17. Procédé selon la revendication 7 ou 8, **caractérisé en ce qu'**une teneur accrue en 1,3-butadiène dans le liquide de pied de la colonne, en particulier de 0,5 à 5 % en poids par rapport au poids total du liquide de pied, de manière préférée de 1 à 3 % en poids, de manière particulièrement préférée de 1,8 % en poids est autorisée, et **en ce que** le liquide de pied est appauvri en 1,3-butadiène dans un évaporateur après avoir été extrait de la colonne, le produit de tête de la colonne à l'état de vapeur étant utilisé de manière préférée en tant que vapeur de stripping.

18. Procédé selon l'une quelconque des revendications 1, 3, 4, 5 ou 6, **caractérisé en ce que** les étapes de procédé I et II sont effectuées dans une colonne à cloison de séparation.

19. Procédé selon la revendication 18, **caractérisé en ce que** l'étape de procédé I est effectuée dans une colonne à cloison de séparation (TK),
- dans laquelle est agencée une cloison de séparation (T) dans le sens longitudinal de la colonne avec formation d'un secteur supérieur de colonne (1) commun, un secteur inférieur de colonne (6) commun, une section d'arrivée (2a, 2b, 4) avec des secteurs de section (2a, 2b, 4) agencés de haut en bas ainsi qu'une section de prélèvement (3a, 3b, 5a, 5b), le secteur de section (5a) de la section de prélèvement (3a, 3b, 5a, 5b) présentant des garnissages réactifs,
- avec apport de la coupe en C₄ (F) dans le secteur médian de la section d'arrivée (2a, 2b, 4), entre les secteurs de section (2b et 4) de cette même section, et apport de l'agent d'extraction (E) dans le secteur supérieur de la section d'arrivée (2a, 2b, 4), entre les secteurs de section (2a et 2b),
- avec apport d'hydrogène (H) en dessous du secteur de section (5a), séparation dans le condenseur (K) de l'hydrogène non converti issu du flux de buées de la colonne à cloison de séparation (TK) d'avec les fractions légères condensables et recyclage par l'intermédiaire d'un compresseur (V) dans le secteur inférieur de la colonne (6) commun, ainsi que
- avec évacuation du flux contenant du 1,3-butadiène (B) issu de la section de prélèvement (3a, 3b, 5a, 5b), de la colonne à cloison de séparation (TK) à un endroit situé entre les secteurs de section (3b) et (5a) et **en ce que**
- le flux (B) est redirigé pour purification par distillation (étape de procédé III).

20. Procédé selon la revendication 19, **caractérisé en ce que** les fractions légères condensées dans le condenseur (K) à partir du flux de buées sont partiellement réexpédiées en tant que reflux vers la tête de la colonne à cloison de séparation (TK), et **en ce qu'**elles sont, par ailleurs, éclusées vers l'extérieur en tant que flux de fractions légères (A).

21. Dispositif de mise en oeuvre du procédé selon la revendication 19 ou 20, **caractérisé en ce que** la colonne à cloison de séparation (TK) présente des garnissages réactifs dans le secteur de section (5a) de la section de prélèvement (3a, 3b, 5a, 5b).

22. Dispositif selon la revendication 21, **caractérisé en ce que** des garnissages réactifs sont contenus de manière supplémentaire dans les secteurs de section supérieurs (3a, 3b) de la section de prélèvement (3a, 3b, 5a, 5b), de manière préférée dans le secteur de section (3b), de manière particulièrement préférée dans les secteurs de section (3a et 3b).
